# EUROPEAN PATENT SPECIFICATION

(11) **EP 3 081 562 B1**
(45) Date of publication and mention of the grant of the patent: **28.02.2018**
(21) Application number: 13899167.4
(22) Date of filing: 09.12.2013
(51) Int. Cl.: C07D 311/92, A61K 31/352, A61P 35/00

(54) **COMPOUND FOR INHIBITING ACTIVITY OF GLUTATHIONE S-TRANSFERASE OMEGA 1 AND PREPARATION METHOD THEREOF, AND PHARMACEUTICAL COMPOSITIONS CONTAINING COMPOUND**
VERBINDUNG ZUR HEMMUNG DER AKTIVITÄT VON GLUTATHION-S-TRANSFERASE OMEGA 1 UND HERSTELLUNGSVERFAHREN DAFÜR SOWIE PHARMAZEUTISCHE ZUSAMMENSETZUNGEN MIT DIESER VERBINDUNG
COMPOSÉ POUR L'INHIBITION DE L'ACTIVITÉ DE LA GLUTATHION S-TRANSFÉRASE OMÉGA 1, SON PROCÉDÉ DE PRÉPARATION ET COMPOSITIONS PHARMACEUTIQUES CONTENANT LE COMPOSÉ

(43) Date of publication of application: 19.10.2016
(73) Proprietor: China Medical University, Taichung City 404 (TW)
(72) Inventor: WU, Yang-Chang, Kaohsiung City (TW); LEE, Kuo-Hsiung, Kaohsiung City (TW); CHANG, Fang-Rong, Kaohsiung City (TW); CHUANG, Da-Wei, Guansi Township Hsinchu County (TW); YANG, Juan-Cheng, Tainan City (TW)
(74) Representative: Lang, Christian
(86) International application number: PCT/CN2013/088871
(87) International publication number: WO 2015/085459

(56) References cited:
- EP-A1- 1 980 248
- WO-A1-94/23715
- TW-A- 200 840 561
- AN-SHEN LIN ET AL: "First total synthesis of protoapigenone and its analogues as potent cytotoxic agents", JOURNAL OF MEDICINAL CHEMISTRY, vol. 50, no. 16, 2007, pages 3921-3927, XP002491004, ISSN: 0022-2623, DOI: 10.1021/JM070363A [retrieved on 2007-07-10]
- KAPIL JUVALE ET AL: "Synthesis and biological evaluation of flavones and benzoflavones as inhibitors of BCRP/ABCG2", EUROPEAN JOURNAL OF MEDICINAL CHEMISTRY, vol. 67, 25 June 2013 (2013-06-25), pages 115-126, XP028710070, ISSN: 0223-5234, DOI: 10.1016/J.EJMECH.2013.06.035

## Description

### Technical Field

The present disclosure relates to a compound for inhibiting glutathione s-transferase omega 1 activity, a pharmaceutical composition containing thereof, and a method for synthesizing the same.

### Description of Related Art

Nowadays, Multi-Drug Resistance (MDR) is believed to be one of the essential topics of pharmaceutical drug research and development on cancer therapy. In addition to the well known channel proteins like p-glycoprotein, MRP1, ABCG2, etc, protein enzymes as metabolic drugs have also been gradually given attention in the field of pharmaceutical drug development. In which, Glutathione S-Transferase (GST) family protein have been playing a critical role.

The GSTs is a phase II metabolic enzyme that favors detoxification of foreign substances of cells; the foreign substances will be linked with glutathione by the GSTs in order to reduce their toxicities. Thus, GST family proteins have been found to be highly expressed in various cancer cells However, both the fundamental and clinical researches indicated that the GSTs have been an important factor involved in drug resistance.

It was found in many studies that one of a protein of the GSTs, GST pi, was essential in association with the acquired resistance to certain anticancer drugs, and thus the GST pi inhibitors have been put under the spotlight by pharmaceutical drug researchers and developers as a main discovering target so as to eliminate such a drug resistance effect. For instance, Telik Biopharmaceutical Company received a great amount of patents of various GST pi inhibitors, some of which have been made as drugs, such as TELCYTA® (Canfosfamide HCl) and TELINTRA® (Ezatiostat HCl, TLK199).

Other isoforms of GSTs, namely GST omega family, such as GST omega 1-1 have been found to be closely associated with drug resistance effects against adriamycin, etoposide, and platinum anticancer drugs. Furthermore, a depletion of GST omega 1-1 in cancer cells circumvents drug resistances against arsenic trioxide, cisplatin, daunorubicin, and etoposide. Nevertheless, inhibitors of GST omega 1 are yet to be discovered nor developed for addressing the drug resistance effects in cancer cells.

Patent document EP 1 980 248 A and the corresponding publication *"*First total synthesis of protoapigenone and its analogues as potent cytotoxic agents" from An-Shen Lin et al. in the JOURNAL OF MEDICINAL CHEMISTRY (Vol. 50, No. 16, 2007, pages 3921-3927) describe the use of some specific flavonoid compounds as potent cytotoxic agents for the treatment of cancer.

### Disclosure of Invention

According to one embodiment of the present invention, a compound with inhibiting glutathione s-transferase omega 1 activity is represented by the following Formula 1: 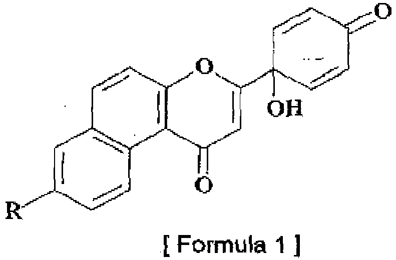 wherein R is isopentyloxy or 3,7-dimethyloctyloxy.

According to another embodiment of the present invention, a pharmaceutical composition includes an effective amount of the compound of the aforementioned Formula 1 and a pharmaceutically acceptable carrier.

According to the present disclosure, a method for synthesizing the compound of Formula 1, includes the following steps. A Friedel-Crafts acylation reaction on 2,6-dimethoxynaphthalene is performed. A halogen reagent is added for removing two methyl groups. An intermediate product having a first protecting group is formed, and then for the intermediate product a Claisen-Schmidt condensation reaction is performed with p-hydroxybenzaldehyde having a second protecting group. A halogen catalyst is added. An acidic solution is further added for removing the first protecting group. An unsaturated carbon chain having halogen is added. Then the second protecting group is removed, and an oxidation reaction is performed by adding a hypervalent iodine compound for obtaining the compound represented by the Formula 1. In one example, the halogen reagent can be Boron tribromide.

In one example, the first protecting group can be methoxymethyl (MOM), and the second protecting group can be benzyl.

In one example, the halogen catalyst can be iodine, and the acidic solution can be hydrochloric acid solution.

In one example, the unsaturated carbon chain can be geranyl bromide.

In one example, the hypervalent iodine compound can be bis-(tri-fluoroacetoxy)-iodobenzene.

According to the above, the compound represented by Formula 1 of the present invention can be used to inhibit GST Omega 1. The method for synthesizing the compound is based on the chemical synthetic route to produce different side-chain derivatives, hence the method has the advantage of mass production. The compound represented by Formula 1 is a derivative of β-naphthalene flavonoids (Protoapigenone), and links the side chain derived from isopentane monomer by ether linkage.

The compound represented by Formula 1 of the present invention targeting GST omega 1 can be a novel anticancer target. Moreover, It has a very high potential for long-term developing as combined therapeutic agents of a variety of anti-cancer drugs.

### Brief Description of The Drawings

The disclosure can be more fully understood by reading the following detailed description of the embodiment, with reference made to the accompanying drawings as follows:

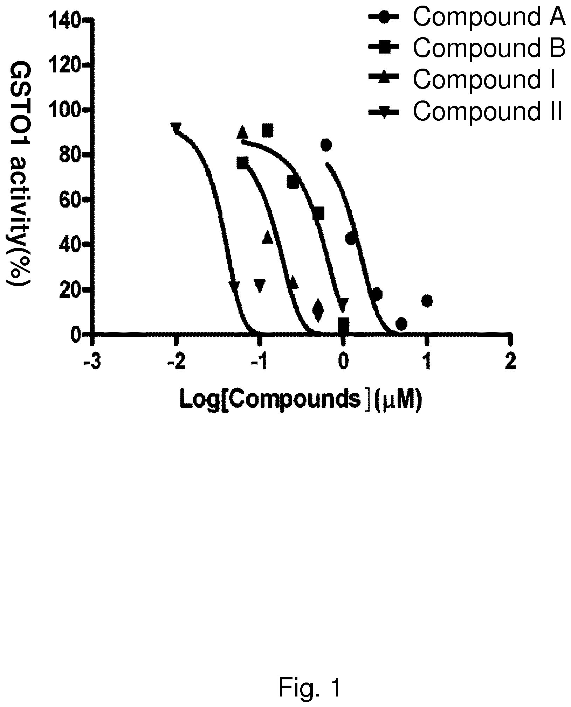

Fig. 1 shows the result of GSTO1 enzyme activity effected by different compounds having different side chains.

### Detailed Description

According to the embodiments of the present nvention, a compound represented by the general Formula 1 is artificially designed and synthesized to be an inhibitory structure binding against the active site of glutathione s-transferase omega 1, which can be applied as a potential pharmaceutical drug in cancer cell cytotoxicity in the future.

### Example 1

For evaluating the in vitro growth inhibitory concentrations (GI₅₀, µM) of the compound represented by Formula 1 of the present invention, human oralepithelial carcinoma cell line (KB), multidrug-resistant nasopharyngeal carcinoma cell line (KB-Vin), human lung adenocarcinoma cell line (A549), and prostate cancer cell line (DU145) were used in the following experiment. The GI₅₀ values of Paclitaxel, an anticancer chemotherapy drug, were used as control. The result of this experiment are shown in Table 1.

The compounds in Table 1 are derivatives of the compound represented by the general Formula 1. The main structure of the compound is 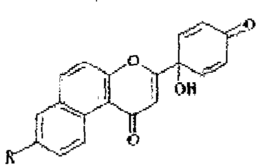 , and R is represented by number 1-19, wherein the side chain represented by number 1-19 is connected to the left end of the main structure to form the compound represented by the general Formula 1.

As shown in Table 1, compounds with side chain No. 11 or No. 12 have better inhibitory effect against the four aforementioned experimental cancer cells when compared with the others having other kinds of side chains.

Further, the compound having side chain No. 11 is an isopentane monomer with an ether linkage, and the GI₅₀ value of such a compound inhibiting against four kinds of experimental cancer cells is 0.2 µM, 0.269µM, 0.382µM, and 0.231µM, respectively. Obviously, the GI₅₀ value of the compound having side chain No. 11 is well and better than the others having other kinds of side chains.

Additionally, the compound having side chain No. 12 has two isopentane monomers on its side chain. The GI₅₀ value of such a compound against four kinds of experimental cancer cells is 0.067 µM, 0.335 µM, 0.233 µM, and 0.065 µM, respectively. This compound has a more significant inhibitory effect against KB cell and DU145 cell.

It is worth to be mentioned that, while increasing the number of isopentane monomers on the side chains to three isopentane monomers, the inhibitory effect against the cancer cells will be significantly reduced (such as the compound having side chain No. 13 in Table 1). As a result, the inhibitory effect of cancer cells inhibited by the compound not only depends on the stereo structure of isopentane monomer, but is also affected by the number of isopentane monomers.

**Table 1. Cancer cell growth inhibitory effect**

| | ***In vitro* cytotoxicity assay : GI₅₀(µM)** | | | | |
|---|---|---|---|---|---|
| No. of side chains | | KB cell | KB-Vin cell | A549 cell | DU145 cell |
| main structure | 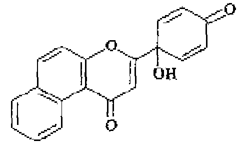 | 0.865 | 0.674 | 0.763 | 0.799 |
| **1** | 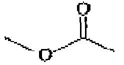 | 0.718 | 0.66 | 0.895 | 0.688 |
| **2** | 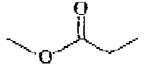 | 0.694 | 0.668 | 0.811 | 0.694 |
| **3** | 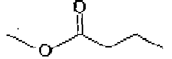 | 0.669 | 0.664 | 0.731 | 0.695 |
| **4** | 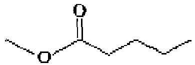 | 0.629 | 0.663 | 0.738 | 0.656 |
| **5** | 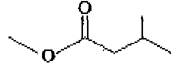 | 0.644 | 0.651 | 0.656 | 0.795 |
| **6** | 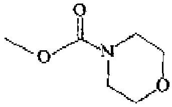 | 0.607 | 0.64 | 0.603 | 0.617 |
| **7** | 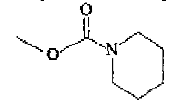 | 0.615 | 0.619 | 0.536 | 0.522 |
| **8** | -OMe | 0.871 | 0.692 | 0.811 | 0.707 |
| **9** | 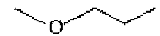 | 0.68 | 0.704 | 0.693 | 0.682 |
| **10** | 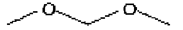 | 0.786 | 0.766 | 0.819 | 0.865 |
| **11** | 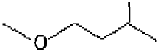 | 0.2 | 0.269 | 0.382 | 0.231 |
| **12** | 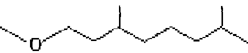 | 0.067 | 0.335 | 0.233 | 0.065 |
| **13** | 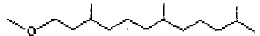 | 1.115 | 1.883 | 427 | 1.262 |
| **14** | 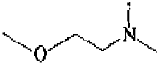 | 0.683 | 0.841 | 0.754 | 0.808 |
| **15** | 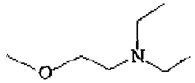 | 0.644 | 1.325 | 0.551 | 0.685 |
| **16** | 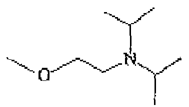 | 0.55 | 0.582 | 0.584 | 0.62 |
| **17** | 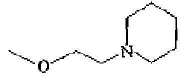 | 0.499 | 0.675 | 0.483 | 0.541 |
| **18** | 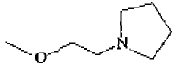 | 0.552 | 1.108 | 0.628 | 0.693 |
| **19** | OH | 0.944 | 0.738 | 0.806 | 0.709 |
| Control (Paclitaxel) | | 4.64 nM | >1000 nM | 3.56 nM | 300 nM |

### Example 2

The following experiment is to prove that the inhibition of the cancer cell growth caused by the compound having side chain No. 11 or No. 12 mentioned above is associated with the inhibitory effect of GSTO1 enzyme activity.

This experiment procedures refers to a GSTO1 substrate assay published by Bachovchin *et al*. in 2009, which uses (S-(4-nitrophenacyl)glutathione; 4NPG) as the specific GSTO1 substrate for evaluating the enzyme activity thereof. First, 100 µl mixture having 2 nM GSTO1, 100 mM Tris (pH 8.0), 1.5 mM EDTA, and 10mM 2-mercaptoethanol were added into each well of an UV-penetrable 96-well culture plate. Besides, 100 µl buffer having 100 mM Tris (pH 8.0), 1-5 mM EDTA, and 10mM 2-mercaptoethanol were used as blank control.

The compound (main structure is represented by Formula 1) to be tested in this assay, having an additional side chain No. 11 and an additional side chain No. 12, respectively, were represented as compound I and compound II. Furthermore, compound A and compound B were controls in this assay, in which compound A does not contain the main structure (Formula 1) of the present disclosure, whereas compound B contains the main structure (Formula 1) of the present disclosure and has a methoxy side chain on the main structure (Formula 1). The structure of compound A, compound B, compound I and compound II are shown in the following:

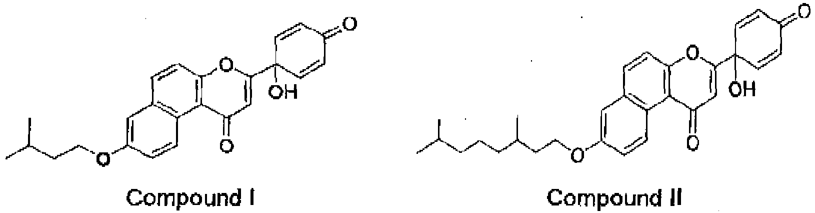

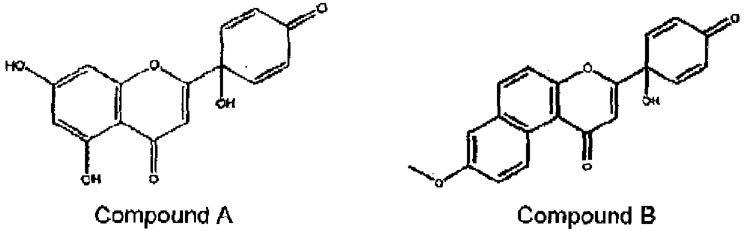

Compound I, compound II, compound A, and compound B were mixed and reacted with dimethyl sulfoxide, respectively, and were added into wells of a 96-well culture plate; the reaction time is 30 min, and the reaction temperature is 25°C. Then, 4NPG were added to each of the aforementioned mixture to a final concentration of 0.5 mM.

The substrate 4NPG can specifically bind to GSTO1, and the structure of the aforementioned compound I and compound II were designed to competitively and structurally bind to the substrate binding site (active site), so that the competitory effect between compound I and 4NPG, or compound II and 4NPG, can be estimated by measuring O.D.₃₀₅ absorbance of each of the mixtures mentioned above in every minute, after calibrating with the blank control.

Fig, 1 shows the result of the GSTO1 enzyme activity effected by different compounds having different side chains. Referring to the result, the inhibitory effect of compound A, which does not contain the main structure (Formula 1) of the present disclosure, and compound B, which contains the main structure (Formula 1) of the present disclosure having side chains without isopentane monomer, against GSTO1 is slight, whereas the compound I and compound II of the present invention remains significant results of competitively binding with GSTO1 against 4NPG. In addition, the inhibitory effect increases as the side chain extends; these results exactly match the result of the aforementioned example 1 (see Table 1).

According to the above results, the compound I and the compound II in the present invention have the significant cytotoxicity effect toward the cancer cells when compared to the compound having other side chains. In addition, it is also confirmed that the structure of the compound I and the compound II can competitively bind with GSTO1 against 4NPG to inhibit the GSTO1 activity.

Therefore, the pharmaceutical composition for inhibiting activity of glutathione s-transferase omega 1 including an effective amount of the compound represented by Formula 1 and a pharmaceutically acceptable carrier can be a novel anticancer target. Moreover, the pharmaceutical composition of the present disclosure has a very high potential for development. It can be long-term developed as combined therapeutic agents of a variety of anti-cancer drugs.

### Example of compound synthesis

The compound with inhibiting glutathione S- transferase omega 1 activity of the present invention can be obtained by conventional method. For example, for 2,6-dimethoxynaphthalene a Friedel-Crafts acylation reaction is performed. Then a halogen reagent is added for removing two methyl groups. An intermediate product having a first protecting group is formed, and then the intermediate product is subjected to a Claisen-Schmidt condensation reaction with p-hydroxybenzaldehyde having a second protecting group. A halogen catalyst is added. An acidic solution is further added for removing the first protecting group. An unsaturated carbon chain having halogen is added. Then the second protecting group is removed, and an oxidation reaction is performed by adding a hypervalent iodine compound for obtaining the compound represented by the Formula 1. The compound (II) of the present invention is used as an example to describe the method for synthesizing the compounds of the present invention. A flow chart of the method for synthesizing the compound II of the present invention is shown in the following:

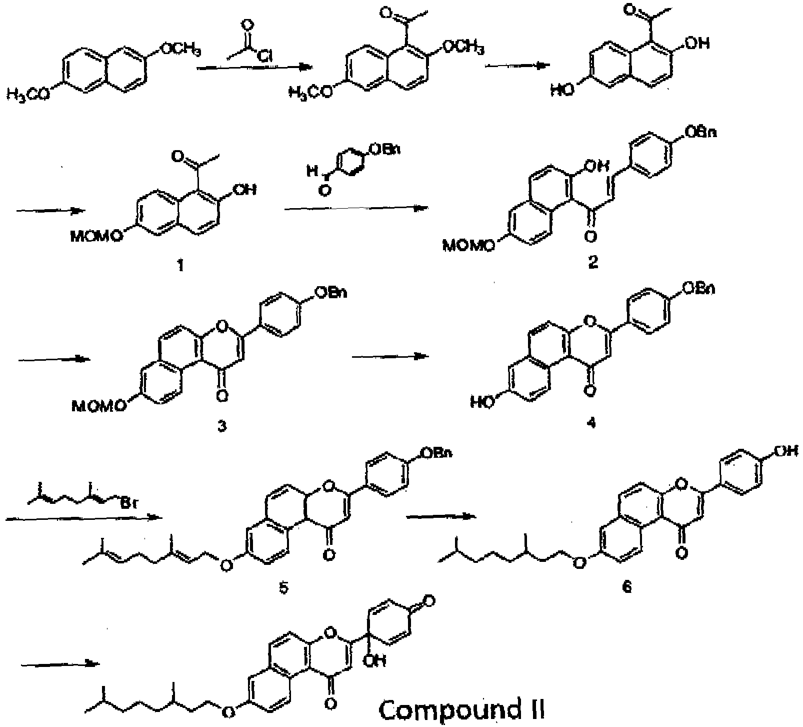

First, anhydrous benzene solution containing 2,6- dimethoxynaphthalene was added to 1.6 N stannic chloride and stirred in a nitrogen environment. Then, 1.5 N acetyl chloride were added dropwise and stirred overnight. Afterwards, benzene was removed by using a rotary evaporator, and then the mixture was extracted with dichloromethane / H₂O. Dichloromethane was eliminated by using a rotary evaporator, then, the extract was separated by an n-hexane / ethyl acetate column. After separation, 1-acetyl-2,6-dimethoxynaphthalene can be obtained, and the yield was 86.5 %.

Afterwards, 1-acetyl-2,6-dimethoxynaphthalene was dissolved in anhydrous dichloromethane, and 6 N tribromoborane were added in a nitrogen environment at -78°C and stirred. After stirring for 2 hours, water was added for removing the remaining tribromoborane. The remaining organic solvents were eliminated by using a rotary evaporator, and then this mixture was separated by an n-hexane / ethyl acetate column. After separation, 1-acetyl-2,6-dihydroxy naphthalene can be obtained, and the yield was 93 %.

1-acetyl-2,6-dihydroxy naphthalene was dissolved in anhydrous dichloromethane, and 2 N of N,N-diisopropylethylamine were added to the mixture on ice continuously until the mixture became transparent. Chloromethyl methyl ether was diluted 40 times with dichloromethane and then added to the mixture dropwise and stirred. After stirring for 2 hours, the remaining organic solvents in the mixture were removed by using a rotary evaporator, and then this mixture was separated by an n-hexane / ethyl acetate column. After separation, compound 1 can be obtained, and the yield was 47.3 %.

Claisen-Schmidt condensation was performed between compound 1 and 4-benzyloxy- benzaldehyde. 3 N 4-benzyloxybenzaidehyde were added into an ethanol solution containing compound 1 and stirred, and then 50 % potassium hydroxide solution were added to the ethanol solution with a volume mixing ratio of 1:1. After stirring in 55°C for 1.5 hours, organic solvents were removed by using a rotary evaporator, and then this mixed solution was separated by an n-hexane / ethyl acetate column. After separation, chalcone compound 2 can be obtained, and the yield was 92.6 %.

Compound 2 was dissolved in a proper amount of pyridine with 2 N iodine added. After heat refluxing overnight, sodium thiosulfate was added and then the whole mixture was extracted with ethyl acetate/water; the organic layer of the extract was removed and then the extract was separated by column chromatography for obtaining β-naphthalene flavone compound 3; the yield was 65 %.

Compound 3 was then dissolved in a proper amount of a dichloromethane solution added with a hydrochloric acid / isopropanol solution with a mixing ratio of 1:10, After stirring overnight, and filtering the precipitate of the mixture, compound 4 was obtained. Anhydrous dimethyl amide solution containing 2 N sodium hydride was dropwise added to a dimethyl amide solution containing compound 4 on ice and in a nitrogen environment until the color of the mixture turns into red. Afterwards, 2N geranyl bromide solution were added into the mixture. Water was added after the mixture was stirred in room temperature for 2 hours, and then the mixture was extracted with ethyl acetate. The remaining organic solvents were removed by using a rotary evaporator, and then this mixture were separated by a dichloromethane / methanol column. After separation, compound 5 can be obtained, and the yield was 94 %.

Compound 5 was dissolved in ethyl acetate, and then 10 % palladium carbon were added. After catalyzing with hydrogen gas overnight, filtering palladium carbon, and removing organic solvents by a rotary evaporator, compound 6 with benzyl removed was obtained, and the yield was 40 %.

Finally, compound 6 was dissolved in a Acetonitrile I water = 15 : 1 solution, and then added with 2 N [bis(trifluoroacetoxy)iodo] benzene for oxidation, compound II having two isopentane monomers on its side chain was obtained, and the yield was 49 %.

The nuclear magnetic resonance (NMR) spectroscopy data of compound II analyzed by NMR spectrometer is shown in the following : 1H NMR (400 MHz, CDCl3) δ = 9.78 (d, J = 9.2 Hz, 1H),7.90 (d,J= 9.2 Hz, 1H), 7.37 (dd, J = 9.2, 2.8 Hz, 1H), 7.31 (d, J = 8.8 Hz, 1H), 7.18 (d, J = 2.4Hz,1H),7.03-7.00(m,3H),6.41(d,J=10.0Hz,2H),5.47(bs,1H), 4.16-4.07 (m,2H), 1.94-1.86(m,1H),1.73-1.61(m,2H),1.59-1.49(m,1H),1.40-1.14 (m, 6H), 0.98 (d, J = 6.4 Hz, 3H), 0.87 (d, J = 6.0 Hz, 6H) ppm; 13C NMR (100 MHz, CDCl3) δ = 185.4, 180.9, 164.0, 158.0, 156.5, 146.7, 135.2, 132.7, 130.0, 128.6, 124.7, 121.2. 117.8, 117.4. 111.6, 108.6, 69.9, 66.7, 39.5, 37.5, 36.3, 30.1, 28.2, 24.9, 22.9, 22.8, 19.9 ppm; IR (KBr) (cm-1): 3294, 2954, 2927, 2869. 1644, 1599, 1514, 1465, 1427, 1410, 1385, 1367, 1245, 1176, 1129, 1058, 1009; HRESI-MS: C29H33O5, calcd. 461.2323, found 461.2328.

## Claims

1. A compound represented by the following Formula 1: 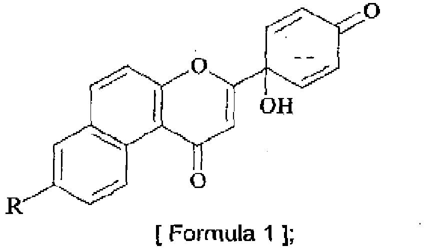 wherein R is isopentyloxy or 3,7-dimethyloctyloxy.

2. A pharmaceutical composition comprising an effective amount of a compound of Formula 1 according to claim 1 and a pharmaceutically acceptable carrier.

## Patentansprüche

1. Verbindung, welche durch die folgende Formel 1 dargestellt wird: 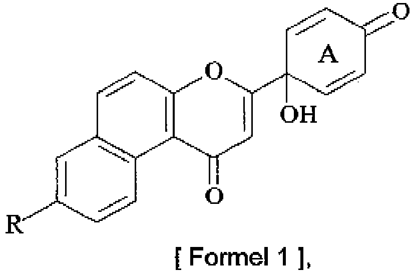 wobei R Isopentyloxy- oder 3,7-Dimethyloctyloxy- ist

2. Pharmazeutische Zusammensetzung, welche eine wirksame Menge einer Verbindung gemäß Formel 1 nach Anspruch 1 und eine pharmazeutisch verträgliche Trägersubstanz umfasst

## Revendications

1. Composé représenté par la formule 1 suivante : 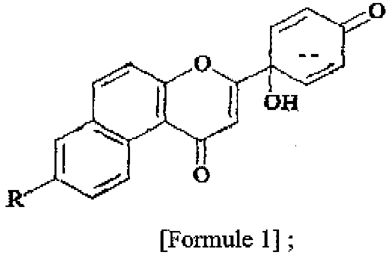 dans lequel R est isopentyloxy ou 3,7-diméthyloctyloxy.

2. Composition pharmaceutique, comprenant une quantité efficace d'un composé de formule 1 selon la revendication 1 et un support pharmaceutiquement acceptable.
